# EUROPEAN PATENT APPLICATION

(11) **EP 1 102 067 A1**
(43) Date of publication of application: **23.05.2001**
(21) Application number: 99922631.9
(22) Date of filing: 01.06.1999
(51) Int. Cl.: G01N 33/573, G01N 33/535, G01N 33/564, C12Q 1/42

(54) **METHOD FOR ASSAYING ANTI-GAD ANTIBODY AND KIT**

(71) Applicant: Kyokuto Pharmaceutical Industrial Co. Ltd, Chuo-Ku, Tokyo 103-0024 (JP)
(72) Inventor: SATO, Yumi Kyokuto Pharmaceu. Industrial Co., Ltd., Takahagi-Shi Ibaraki 318-0004 (JP); WADA, Naruhito Kyokuto Pharmaceu. Ind. Co., Ltd., Takahagi-Shi Ibaraki 318-0004 (JP); KOJIMA, Masaharu Kyokuto Pharmaceu. Ind. Co., Ltd., Takahagi-Shi Ibaraki 318-0004 (JP); TANNO, Kazunobu Kyokuto Pharmaceu. Ind. Co., Ltd., Takahagi-Shi Ibaraki 318-0004 (JP)
(74) Representative: Wibbelmann, Jobst, Dr., Dipl.-Chem.
(86) International application number: JP9902925
(87) International publication number: WO0073800

(57) **Abstract**

The present invention provides a safe and outstanding method for detecting or measuring an anti-GAD antibody and a kit therefor, which shows a high sensitivity and specificity that are comparable to those of radioimmunoassays. More specifically, the present invention provides a method for detecting or measuring an anti-GAD antibody comprising contacting a sample with a carrier on which a recombinant GAD antigen is immobilized, binding said antigen and said antibody in the sample to form a complex, and detecting said complex by a chemiluminescent dioxetane having a group that can be cleaved with an enzyme and an antibody labeled with an enzyme that can cause chemiluminescence by cleaving said group, and a kit for the method.

## Description

### Field of the Invention

The present invention relates to a method and kit for detecting antibodies against glutamic acid decarboxylase (hereafter often called "GAD"). Particularly, the present invention relates to a method and kit for detecting and quantifying human anti-GAD autoantibodies useful for the diagnosis of diabetes.

### Background Art

Glutamic acid decarboxylase (hereafter often called "GAD") is an enzyme involved in the production of γ-aminobutyric acid (GABA) from glutamic acid and is localized in islet cells (mainly β cells) of the pancreas, the brain, and so on. There are at least two molecular species of GAD known in the pancreas and the central nervous system: GAD67, with a molecular weight of about 67,000 kD, and GAD 65, with a molecular weight of about 65,000 kD.

Recently, it has been found that GAD is identical to what is generally called the "64 kD autoantigen" that has been conventionally detected in diabetes patients. GAD has been identified as one of those autoantigens that are associated with insulin-dependent diabetes mellitus (hereafter often called "IDDM"). Although anti-GAD antibodies are detected among diabetes patients, particularly IDDM patients, they are also present in the sera of non-insulin dependent diabetes mellitus (hereafter often called "NIDDM") patients, and some of those patients proceed to develop IDDM. Also, in the case of slowly progressive IDDM (hereafter often called "SPIDDM"), anti-GAD antibodies are detected before the expression of the clinical signs of diabetes. Further, anti-GAD antibodies may be detected in diabetes called LADA, which has been reported recently as an intermediate type between IDDM and NIDDM (Namai et al., Diabetes Frontier, 6, No. 4, p. 404, 1995). Therefore, by detecting anti-GAD antibodies, the diagnosis or prediction of diabetes, particularly IDDM, is possible.

Diagnostic methods based on the detection of anti-GAD autoantibodies have been known (for example, see WO92/04632 and WO92/05446). In these references, a radioimmunoassay (RIA) and an enzyme-linked immunosorbent assay (ELISA) and so on are listed as particular detection methods. In such assays, the selection of the antigen and detection means is important in terms of the sensitivity and specificity.

Anti-GAD antibodies in the sera of patients who have diabetes (particularly insulin-dependent diabetes mellitus) are known to react mainly with GAD65 (see Ujihara et al., Diabetes, 43, p. 968, 1994). Natural GAD65 is extracted and purified mainly from the porcine brain. However, when used as the antigen it reduces the specificity of the assay, because such a preparation is contaminated with GAD67, and accurate measurements cannot be obtained. Thus, it is expected that the usefulness of the assay will become higher by using a recombinant GAD65, i.e., GAD65 that is prepared by genetic engineering (see, for example, WO92/05446 and WO94/18568), rather than a natural one.

However, GAD65, regardless of whether it is a natural or recombinant one, is known to have a poor purification yield due to its high hydrophobicity (see Bu et al., Proc. Natl. Acad. Sci. USA, 89, p. 2115, 1992; and Bu et al., Genomics, 21, p. 222, 1994). A dominant epitope in a GAD65 molecule is located from the center to the C-terminal region, while the highly hydrophobic N-terminal region contributes poorly to the immunoreactivity. In contrast, the
N-terminal region of GAD67 is less hydrophobic compared to that of GAD65. Thus, to improve yields without disturbing the immunoreactivity of GAD65 by reducing the hydrophobicity of the molecule, Mackay et al. of Monash University, Australia, prepared a recombinant molecule in which the N-terminus of GAD65 was replaced with that of GAD67 (the recombinant hybrid GAD67/65 antigen) (see Law et al., J. Biotechnol., 61, p. 57, 1998). This molecule has been shown to react with human anti-GAD autoantibodies in an immuno-precipitation assay and thus to be useful for the diagnosis of IDDM and the detection of pre-clinical conditions. However, Mackay et al. has not reported a detection system using this antigen that can replace radioimmunoassays.

In WO92/04632 and WO92/05446, it is stated that as the label for the detection of an anti-GAD antibody, coloring dyes, chemiluminescent compounds, bioluminescent compounds, colloidal metals, fluorescent dyes, etc., can be used. However, there is no specific description of a detection method using them. Further, the currently-sold human anti-GAD autoantibody assay reagents are only those based on radioimmunoassays. In using a reagent based on a radioimmunoassay there are problems or restrictions, as below, associated with handling radioisotopes:
1) facilities that can use the reagents are restricted to those that are allowed to handle radioisotopes;
2) radioisotopes and related reagents are costly; and
3) in relation to No. 1 above, special facilities for the prevention of exposure or contamination and the treatment of radioactive wastes, etc., are required.

Thus, to conduct assays without the restrictions caused by those problems, there is a need for safe assay technology that do not use radioisotopes.

### Summary of the Invention

The object of the present invention is to enable a (safe) assay for anti-GAD antibodies that has a performance that is comparable to radioimmunoassays.

The present inventors completed the present invention by finding that an anti-GAD antibody could be detected at a sensitivity and specificity comparable to those of radioimmunoassays by using a recombinant GAD antigen and a chemiluminescent dioxetane.

An embodiment of the present invention is an enzyme-linked immunosorbent assay (ELISA) for detecting or measuring an anti-GAD antibody comprising contacting a sample with a carrier on which a recombinant GAD antigen is immobilized, binding said antigen and said antibody in the sample to form a complex, and detecting said complex by a chemiluminescent dioxetane having a group that can be cleaved with an enzyme and an antibody labeled with an enzyme that can cause chemiluminescence by cleaving said group.

Another embodiment of the present invention is an enzyme-linked immunosorbent assay kit for detecting an anti-GAD antibody comprising a carrier on which a recombinant GAD antigen is immobilized, a chemiluminescent dioxetane having a group that can be cleaved with an enzyme, and an antibody labeled with an enzyme that can cause chemiluminescence by cleaving said group.

In a preferred embodiment of the method or kit of the invention, used are a carrier on which a recombinant hybrid GAD67/65 antigen is immobilized, a dioxetane that has a phosphate group cleavable with an alkaline phosphatase and produces chemiluminescence upon the cleavage of the group, especially CDP-Star ® or AMPPD ®, and an alkaline phosphatase-labeled anti-human immunoglobulin G (IgG) antibody.

### Brief Description of the Drawings

Figure 1 is a flowchart showing an example of the procedure for preparing a recombinant hybrid GAD67/65 antigen-immobilized plate.
Figure 2 is a flowchart showing a specific example of a human anti-GAD autoantibody assay of the present invention.
Figure 3 is a diagram showing the relationship between antibody concentrations and the dilutions of samples; samples containing high concentrations (samples A, B, and C; Fig. 3a) and medium-to-low concentrations (samples D and E; Fig. 3b) of anti-GAD antibodies (i.e., anti-GAD antibody positive sera) were diluted with a sample diluent.
Figure 4 is a diagram of the effects of interfering substances in samples on the method of the present invention. The interfering substances are: 4a = hemoglobin; 4b = bilirubin C; 4c = bilirubin F, 4d = ascorbic acid; and 4e = chyle.
Figure 5 is a diagram showing the sensitivity of detection when human anti-GAD antibodies were measured with a kit of the present invention using the reagents of Example 1.

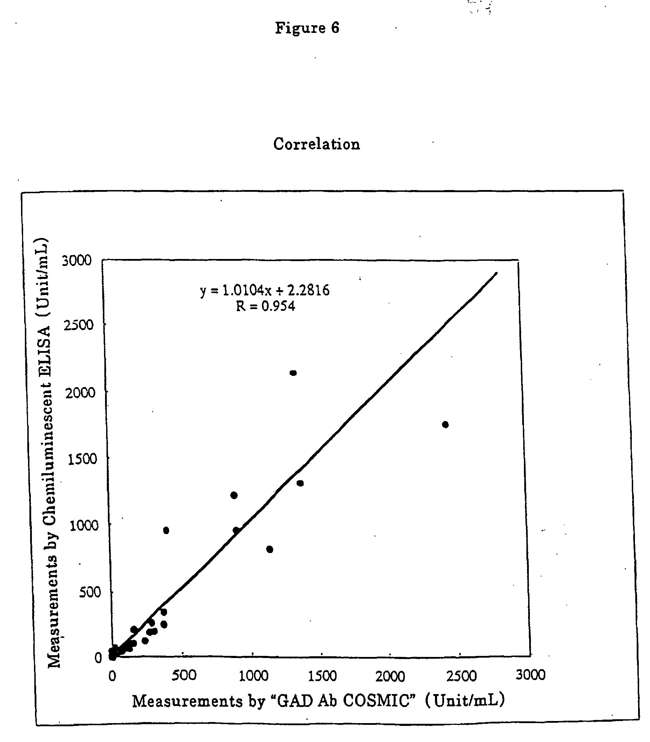

Figure 6 is a diagram showing the correlation between measurements of human anti-GAD autoantibody concentrations using a kit of the present invention with the reagents of Example 1 and those using a conventional radioimmunoassay kit.

### Detailed Description of the Invention

### 1. ELISA

General methods of ELISA are well-known. One of those methods is as outlined below. An antigen which is the target of the antibody to be detected (human anti-GAD antibody), i.e., a GAD antigen, is bound to a carrier, with which carrier a sample is reacted (contacted). When anti-GAD antibodies are present in the sample, they will bind to the carrier via the GAD antigen. Then a labeled anti-human immunoglobulin antibody will bind to the anti-GAD antibody bound to the carrier and the labelled material is detected. Thus human anti-GAD antibodies can be measured.

A further explanation is now provided for an exemplifying case in which a GAD (antigen)-immobilized plate is used. A specimen containing an anti-GAD antibody (a serum, plasma, blood, urine, saliva, lacrimal fluid, or lymph fluid and so on, appropriately diluted or undiluted) is added to the plate to allow the binding of the GAD antibody to the GAD antigen. Thereafter, the plate is washed to remove any component that did not bind to the GAD antigen. Then an appropriate amount of an enzyme-labeled anti-human immunoglobulin antibody is added to allow binding to the GAD antigen. Then the plate is washed to separate and remove any excess enzyme-labeled anti-human immunoglobulin antibody that did not bind to the anti-GAD antibody. Further, by adding an appropriate enzyme substrate and measuring the final product of the enzyme reaction, the concentration of the anti-GAD antibody in the specimen can be measured. The concentrations of anti-GAD antibodies can similarly be measured when GAD-immobilized microparticles etc. are used as the carrier.

As an alternative method, competitive ELISA is also well-known. In this case, an enzyme-labeled anti-GAD antibody is present with the anti-GAD antibody in the specimen, so that they will compete for the binding to the GAD antigen. Then the decrease in the degree of binding of the labeled anti-GAD antibody caused by the binding of the anti-GAD antibody in the specimen is measured.

The ELISA and kit of the present invention enable a detection of an anti-GAD antibody at a high sensitivity and specificity that is comparable to a radioimmunoassay, by using the reagents described below in conventional ELISA as above.

### 2. Reagents etc. used in the present invention

### 2.1. Recombinant GAD antigen

The recombinant GAD antigen used in the present invention can be a GAD antigen prepared by genetic engineering that comprises all or a part of the GAD65 antigenic polypeptide. Those comprising at least a part of amino acids 244 to 570, preferably at least amino acids 244 to 435 and 451 to 570, of GAD65, is desirable, since a dominant epitope in the GAD65 antigen is located from its center to the C-terminal portion. The recombinant hybrid GAD67/65 antigen is particularly preferred for the ease of purification and its yield. The "recombinant hybrid GAD67/65" as used herein refers to a hybrid molecule, prepared by genetic engineering, in which a part of GAD67 and a part of GAD65 are fused to constitute one molecule. An example is the one described by Law et al., J. Biotechnol., 61, p. 57, 1998. This is a fusion of amino acids 1 to 101 from human GAD67 and amino acids 96 to 585 from human GAD65. This can be produced in yeasts, E. coli, insect cells, and so on, and readily purified by using an affinity column.

A recombinant GAD antigen can also be one of those produced by myelomas, such as the one described in a patent specification by Toso, Inc. (Japanese Provisional Patent Publication (Kokai) H9-313183).

A recombinant GAD antigen can be used by binding it to a carrier. Numerous types of carriers suitable for ELISA are known, including, for example, glass, polystyrene, polyvinyl chloride, polypropylene, polyethylene, polycarbonate, dextran, nylon, amylose, natural and modified cellulose, polyacrylamide, agarose, and magnetite. Forms of the carriers include, for example, a microtiter plate and microparticles. Commercially available carriers include "FluoroNunc™ Modules" from Nalge Nunc International and "Opaque Strip Well" from Corning Costar etc. as plates; "Polybead Polystyrene Microspheres, Polystyrene Paramagnetic Microspheres" from Polyscience, Inc. and "Estapor® (magnetic particles and non-magnetic particles)" etc. from Prolabo as microparticles, and so on.

The carrier to which a recombinant GAD antigen used in the invention is immobilized, i.e., bound, can be prepared by binding a recombinant GAD antigen to the carrier directly or via other material according to a conventional method in the art.

Methods for direct binding include a method in which an appropriate concentration of a GAD solution is added to the wells of a plate and kept standing overnight.

Methods for binding via other materials include those binding via a bridging agent or an anti-GAD antibody. For example, a GAD antigen can be bound to a plate by adding to the wells of the plate an appropriate amount and concentration of an anti-GAD antibody solution, keeping it standing overnight, and then adding to the wells of the plate an appropriate amount and concentration of a GAD solution and keeping it standing overnight.

The amount of the recombinant GAD antigen to be bound to a carrier may be one that will not cause saturation of the light intensity of the chemiluminescent substance used for the detection. Specifically, for example, about 0.5 to 2.0 µg/well of such hybrid GAD 67/65 antigen is preferred. This amount is measured by the Bradford method, and this amount of protein is acting as the GAD antigen.

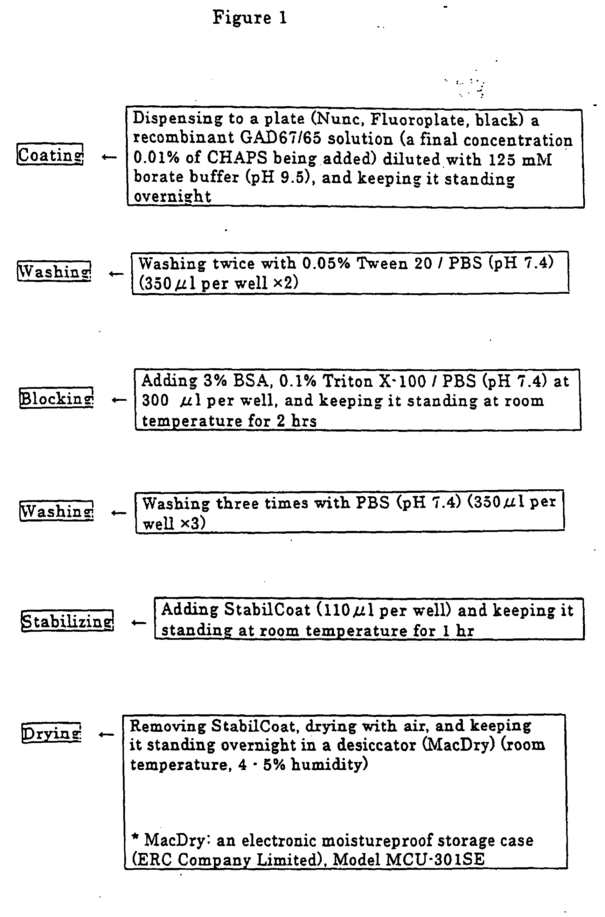

Figure 1 indicates an example of the procedure to prepare a GAD-immobilized carrier by direct binding that can be used in the present invention. In the figure, PBS refers to the phosphate-buffered saline. A carrier to which a recombinant GAD antigen is immobilized may be prepared when used, or prepared in advance and stored appropriately. The conditions for the storage etc. are well known to those skilled in the art. For example, a carrier to which a recombinant GAD antigen is immobilized can be stably stored at 4 °C or ―20 °C etc. with or without drying.

### 2.2. Chemiluminescent dioxetanes

As a chemiluminescent dioxetane used in the present invention, those disclosed in WO88/00695, Japanese Provisional Patent Publication (Kokai) H08-13406 and related patent applications can be used. For example, dioxetanes expressed as the formula: 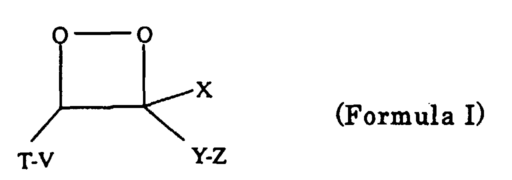
(wherein T is one or more of C [carbon number] 1 to C7 alkyls, C6 to C12 cycloalkyls which may be substituted with a heteroatom group, or polycycloalkyls which have a spiro-union with the four-member moiety of a dioxetane; V is a hydrogen or group that can be cleaved with an enzyme; Y is a fluorescent chromophore; X is a hydrogen, linear, or branched C1 to C7 alkyl, linear or branched C1 to C7 heteroalkyl, an aryl having at least one ring, a heteroaryl having at least one ring, C3 to C7 cycloalkyl, a C2 to C7 heterocycloalkyl, an aralkyl having at least one ring, an alkylaryl having at least one ring, or a group that can be cleaved with an enzyme; Z is a hydrogen, a hydroxyl group, or a group that can be cleaved with an enzyme; and at least one of V, X, and Z is a group that can be cleaved with an enzyme).

As a fluorescent chromophore, anthracene, rhodamine, fluorescein, eosin, coumarin, erythrosine, acridine, pyrene, stilbene, naphthalene, nitrobenzoxadiazole, quinoline, acid acridine, carbazole, fluorescent cyanines, carbocyanine, pyridinium, oxonols, rezorufines, and hydroxyrezorufines, and derivatives thereof are included.

Preferably, T is a polycycloalkyl group, with an adamantyl group that may be substituted, with a halogen, methoxy, isopropyloxy, or normal butyloxy group particularly preferred. Preferably, X is a C1 to C7 alkyl group that is bound to oxygen, such as a methoxy or ethoxy group.

Examples of those groups that can be cleaved by an enzyme include phosphate, acetate, carboxyl, 1-phospho-2,3-diacylglyceride, β-D-xyloyde, β-D-fucoside, 1-thio-D-glucoside, adenosine triphosphate analogues, β-D-adenosine diphosphate analogues, adenosine monophosphate analogues, β-D-galactoside, α-D-galactoside, α-D-glucoside, β-D-glucoside, α-D-mannoside, β-D-mannoside, β-D-fructofuranoside, β-D-glucosido uronate, P-toluenesulfonyl-L-arginine dye ester, and P-toluenesulfonyl-L-arginine dye amido. Preferably, a group that can be cleaved with an enzyme is a phosphate group.

Thus, more particularly, a dioxetane expressed by the formula: 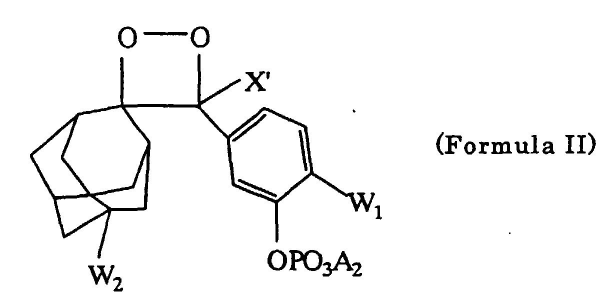
(wherein X' is a methoxy, ethoxy, propoxy, or butoxy; W₁ is a hydrogen or halogen; W₂ is a hydrogen, halogen, methoxy, isopropyloxy, or normal butyloxy; W₁ and W₂ may be the same or different; and A is an alkali metal).

Particularly, disodium 2-chloro-5-(4-methoxyspiro{1,2-dioxetane-3,2'-(5'-chloro)-tricyclo[3.3.1.1^{3,7}] decan}-4-yl)-1-phenyl phosphate of the formula: 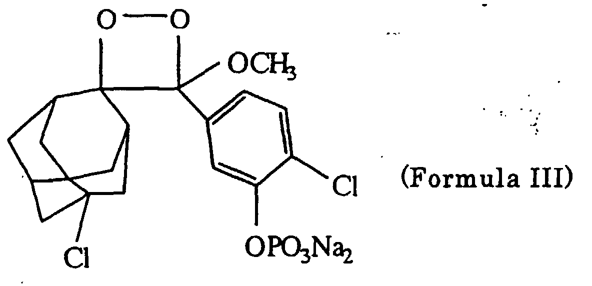 or disodium 3-(4-methoxyspiro{1,2-dioxetane-3,2'-tricyclo [3.3.1.1^{3,7}] decan}-4-yl) phenyl phosphate, i.e., the compound also called 3-(2-spiroadamantane)-4-methoxy-4-(3''-phosphoryloxyphenyl-1,2-dioxetane), of the formula: 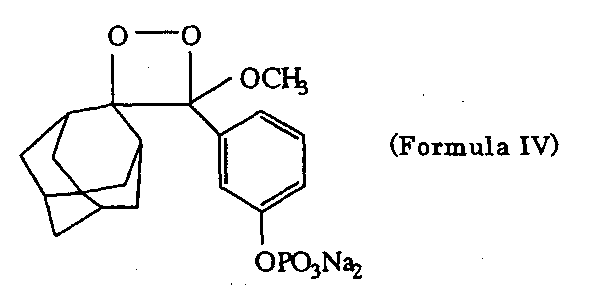 is preferred. The compounds of formulae III and IV are available from Tropix, Inc. under the tradenames "CDP-Star" (a registered trademark) and "AMPPD" (a registered trademark), respectively. By using CDP-Star or AMPPD as the substrate for measuring anti-GAD antibodies, it is possible to remarkably and rapidly obtain a light intensity peak and to keep the light intensity stable for a long time.

Further, to increase the intensity of the chemiluminescence of dioxetanes, it is preferred to use an enhancer, such as a quaternary ammonium homopolymer, particularly poly(benzyltributyl) ammonium chloride or a composition containing the same (Sapphire II or Emerald II; both are tradenames and available from Tropix, Inc.).

### 2.3. Enzyme-labeled antibody

Enzymes used in the present invention that can cause chemiluminescence by cleaving a specific group of a dioxetane include alkaline or acid phosphatases, esterases, decarboxyrases, phospholipase D, β-xylosidase, β-D-fucosidase, thioglucosidase, ATPases, ADPases, 5'-nucleotidase, β-D-galactosidase, α-D-galactosidase, α-D-glucosidase, β-D-glucosidase, α-D-mannosidase, β-D-mannosidase, β-D-fructofuranosidase, β-D-glucosiduronase, and trypsin. Phosphatases are particularly preferred.

An antibody labeled with such an enzyme may be one of those commercially available, or prepared by a known method using an appropriate enzyme and antibody. The enzyme may be bound to the antibody directly or via another material or other materials. Those other materials include, for example, avidin and biotin or streptoavidin and biotin.

When the sample is derived from a human, an intact anti-human immunoglobulin antibody (IgG etc.) or any fragment of it, such as Fab, F(ab')₂, etc., may be used as the antibody. When an enzyme-labeled antibody is prepared by oneself, those anti-human IgG antibodies that bind the H chain, L chain, γ chain, or Fc portion of human IgG are preferably used. In those situations, the ratio of the number of molecules of the enzyme to the anti-human IgG antibody is as follows: enzyme: anti-human IgG antibody = 0.5 to 10:1, preferably 2 to 5:1.

As the enzyme-labeled antibody used in the present invention, alkaline phosphatase-labeled anti-human IgG antibodies are particularly preferred. These may be commercial products or prepared by oneself using an alkaline phosphatase and an anti-human IgG antibody.

Normally, an alkaline phosphatase-labeled anti-human IgG antibody is contained in a stock solution containing a stabilizer. The stabilizer includes, for example, bovine serum albumin, glycerol, trehalose, StabilCoat (AR Brown), and so on.

### 2.4. Other reagents

As the sample for which the presence of an anti-GAD antibody is detected or its concentration is measured by the method or kit of the present invention, a biological sample, particularly serum, plasma, blood, urine, saliva, lacrimal fluid or lymph fluid, and so on, diluted or undiluted, is used.

Any solution containing an anti-GAD antibody may be used as the anti-GAD antibody standard or positive control used in the assay. Particularly, when the sample is a serum, it is preferred to use an anti-GAD antibody positive patient serum from an insulin-dependent or non-dependent diabetes patient. Usually, the same solution as that of the positive control, but which solution contains no or substantially no anti-GAD antibody, is used as the anti-GAD antibody negative control. Examples include an anti-GAD antibody negative human serum.

As the sample diluent, a phosphate buffer, Tris-HCl buffer, borate buffer, Veronal buffer, or a saline containing any of them, etc., can be used. A serum, preservative, and so on may be added to the sample diluent. For example, the one described in Table 1 below may be suitably used as a sample diluent.

The compositions and methods for preparation, etc., of solutions such as washing buffers, blocking solutions, and buffers used in ELISA are well known to those skilled in the art. Skilled artisans can freely select and use what are suitable for the practice of the present invention.

### 2.5. The kit of the present invention

The reagents, etc., used in the assay of the present invention may be provided in the form of a kit. The kit of the present invention comprises as its components at least a carrier to which a recombinant GAD antigen is immobilized, a chemiluminescent dioxetane having a group that can be cleaved with an enzyme, and an antibody labeled with the enzyme that can cause chemiluminescence by cleaving the group. Further, the kit may also comprise one or more components that are used in the practice of the present invention, such as a sample diluent, anti-GAD antibody standards or positive and negative controls, a washing solution, etc.

### 3. Procedure of the assay of the invention

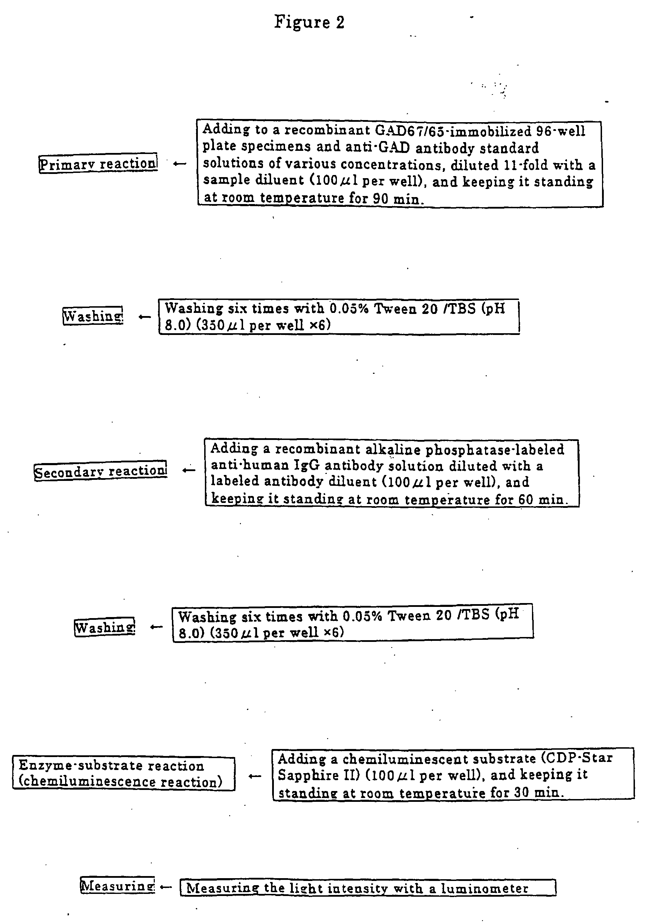

Now the anti-GAD antibody assay of the present invention is described step-by-step. Specifically, explained as a typical example is a method in which the light intensity is measured according to the procedure in Figure 2 and the anti-GAD autoantibody concentration is calculated based on the measurement.

First, in the primary reaction, an anti-GAD antibody in a sample is bound to a recombinant GAD antigen immobilized on a carrier. The sample used is an undiluted or appropriately diluted specimen (biological sample). When measured quantitatively, standard solutions containing known concentrations of an anti-GAD antibody (positive controls) are simultaneously tested with necessary negative controls. The reaction temperature can generally be 20 to 37 °C. For ease of operation and so on, room temperature is preferred. When quantitatively measuring using a microtiter plate, all of the primary reaction and the secondary and enzyme-substrate reactions (including the luminescence reaction) described below are preferably conducted at room temperature. This is because in an assay using a microtiter plate, the edge effect, i.e., the phenomena in which the reaction in the wells located at the top and bottom edges and both sides of a plate proceeds faster than that in central wells, is often observed when the plate is externally heated. So, operations such as heating, etc., should be avoided. As for the reaction time, a time that is as short as possible and that provides a stable reaction rate is desired. The reaction time is generally about 20 to 120 minutes, though those skilled in the art can appropriately determine the optimum reaction time depending on the amounts of the immobilized antigen and the antibody in the sample, and so on. For example, the primary reaction is preferably designed to last 60 to 120 minutes, particularly about 90 minutes, because the reaction rate is sufficiently slow after 90 minutes at the anti-GAD antibody concentration of 625 Unit/L (the unit of concentration used by Mackay et al. at Monash University) and the light intensities among anti-GAD autoantibody concentrations are sufficiently distinct.

Washing is conducted using a solution such as a saline buffered with phosphate, borate, Tris-HCl, etc., a washing buffer containing a detergent, and so on. Washing is for separating and removing components that did not bind to the carrier. Those skilled in the art can appropriately determine the compositions and amounts of washing solutions, and the time and repetitions, etc., of washing operations. For example, an appropriate amount of a Tris-HCl buffer or phosphate-buffered saline (pH 7.2 to 8.0) containing a low concentration (e.g., 0.05 to 0.5 %) of Tween 20 is added to the carrier and removed in one to six repetitions, to wash the carrier.

Then the secondary reaction is conducted. More specifically, an enzyme-labeled antibody is added to the carrier after washing to allow binding to an anti-GAD antibody. The reaction temperature is as described above for the primary reaction. For the reaction time, generally 15 to 90 minutes, particularly about 60 minutes, is preferred, so that the light intensity at 0 Unit/L of anti-GAD autoantibody concentration is as low as possible, yet samples are measurable in a short time.

The washing after the secondary reaction is conducted similarly to that after the primary reaction. More specifically, the carrier is washed to separate and remove excess enzyme-labeled antibodies that did not bind to the anti-GAD antibody.

The enzyme-substrate reaction is conducted by adding an appropriate enzyme substrate to the carrier after washing and incubating them for a predetermined time. The appropriate reaction time and temperature may vary depending on the type, amount, composition, etc., of the enzyme, substrate, buffer, etc., that are used, but those skilled in the art can adequately determine them. For example, in the assay and kit of the present invention using the reagents described in Example 1 below, the enzyme-substrate reaction is preferably conducted at a reaction temperature of 25 °C and for a reaction time of about 30 minutes, because the reaction at about 25 °C reaches a plateau in 30 minutes. For example, the enzyme-substrate reaction is conducted by adding to wells of a microtiter plate a chemiluminescent dioxetane (e.g., CDP-Star Sapphire II from Tropix, Inc.) in an amount of 100 µl/well at a concentration of 0.4 x 10⁻³ mol/L and allowing it to stand for 30 minutes at room temperature.

The measurement is conducted by counting with a luminometer, etc., the light emission (intensity) of the enzyme reaction product finally produced by the enzyme-substrate reaction. As described below, the amount of the antibody and the light intensity are directly proportional. Thus the concentration of the anti-GAD antibody in the specimen can be measured. The measurement of a specimen by a luminometer (relative light intensity; RLU) can be converted into the concentration of the anti-GAD antibody by using a graph, i.e., a calibration curve, indicating the relationship between the counted values of anti-GAD antibody standard solutions (RLU) and the antibody concentrations in the standard solutions.

Anti-GAD antibody concentrations are similarly measured when GAD-immobilized microparticles, etc., are used as the carrier.

The present invention enables a safe and highly sensitive measurement of a human anti-GAD antibody. According to the present invention, provided are a safe high-performance method for detecting and measuring an anti-GAD antibody and a kit therefor that show a high sensitivity and specificity comparable to radioimmunoassays. The method and kit of the present invention enable a precise quantification of an anti-GAD antibody without being affected by interfering substances in a biological sample. Thus they are useful for the diagnosis or prediction of diabetes, particularly IDDM.

### Examples

The present invention is further illustrated by the examples below.

### Example 1: Preparation of reagents and a GAD antigen-immobilized plate used for the anti-GAD antibody assay of the present invention

Various reagents with the compositions listed in Table 1 were prepared or obtained.

**Table 1**

| Reagent | Composition |
|---|---|
| GAD antigen | Recombinant hybrid GAD67/65 antigen (obtained from Monash University; described in J. Biotechnol, 61, p. 57 (1998)), 10 µg/ml, in 125 mM borate buffer (pH 9.5) containing 0.01% |
| | CHAPS |
| | (Coating was at 1 µg/well) |
| | (The amount of protein is a value according to the Bradford method.) |
| Blocking solution | PBS (pH 7.4) containing 3%(w/v) bovine serum albumin (BSA), and 0.1%(w/v) Triton X-100 (polyoxyethylene (10) octylphenylether; Wako Pure Chemical Industries, Ltd.) |
| Sample diluent | PBS (pH 7.4) containing 30%(v/v) goat serum (inactivated by heating at 56°C, 30 minutes), 20%(w/v) glycerol and 0.1%(w/v) sodium azide |
| Anti-GAD antibody standard solutions | Patient serum with a high anti-GAD antibody value, of 0, 6.3, 25, 50, 100, 250, 500, and 1000 (unit/L) |
| | (diluted with an anti-GAD antibody negative serum; containing 0.1%(w/v) sodium azide) |
| Labeled antibody diluent | 30 mM Tris-HCl (pH 7.4) buffer containing 150 mM NaCl, 1%(w/v) BSA, 1 mM L-glutamic acid, 1 mM S-2-aminoethylisothiouronium bromide, 0.1%(w/v) Nonion HS 210 (tradename: NOF Corporation), and 0. 1%(w/v) sodium azide |
| Labeled antibody solution | Stock solution (50 mM Tris-HCl (pH 8.5) containing 150 mM NaCl, 1%(w/v) BSA, 1 mM MgCl₂, 20%(w/v) glycerol, and 0.1%(w/v) sodium azide) containing 0.8 x 10⁻⁶ mol/L recombinant ALP-labeled goat anti-human IgG (Fc) antibody |
| Washing solution (x20) | 600 mM Tris-HCl (pH 8.0) containing 3M NaCl, 1%(w/v) Tween 20, and 2%(w/v) sodium azide |
| Chemiluminescent substrate solution | 0.4 x 10⁻³ mol/L CDP-Star Sapphire II (obtained from Tropix, Inc. ) |

| | |
|---|---|
| * Composition of PBS (pH 7.4): 137 mM NaCl, 8.1 mM disodium hydrogenphosphate 12 hydrate, 2.7 mM potassium chloride, and 1.5 mM potassium dihydrogenphosphate. | |

A GAD antigen-immobilized plate was prepared by immobilizing a GAD antigen on a plate. Specifically, it was done according to the method shown in Figure 1. To wells of a microtiter plate (Nalge Nunc International, Fluoro Plates, black), 125 mM borate buffer (containing 0.01% CHAPS, pH 9.5) containing the recombinant hybrid GAD 67/65 at a concentration of 10 µg/ml was dispensed at 100 µl/well and kept standing at 4 °C overnight. The wells were washed twice with 350 µl/well of PBS (pH 7.4) containing 0.05% Tween 20. Then, to the wells, 300 µl/well of PBS (pH 7.4) containing 3% bovine serum albumin (BSA) and 0.1% Triton X-100 was added as the blocking solution and the plate was kept standing at room temperature for two hours for blocking. The wells were then washed each of three times, each time with 350 µl/well of PBS (pH 7.4). A StabilCoat solution (AR Brown) was added to each well at 110 µl/well and kept standing at room temperature for one hour to stabilize the immobilized antigen. After removing the StabilCoat solution and the plate being air-purged, the plate was kept standing in a desiccator (an electronic moistureproof storage case MacDRY, ERC Company Limited, Model MCU-301SE) to dry overnight at room temperature, at 4 to 5% humidity.

In the following experiments, the blocking solution, sample diluent, anti-GAD antibody standard solutions, labeled antibody diluent, and chemiluminescent substrate solution were used without dilution. When used, the labeled antibody solution was used at a 100-fold dilution with the labeled antibody diluent. When used, the washing buffer was used at a 20-fold dilution with purified or distilled water.

### Example 2: Confirmation of dilution linearity

Specimens with high (sample A=900 Unit/L, sample B=380 Unit/L, sample C=230 Unit/L), medium (sample D=115 Unit/L) and low (sample E=43 Unit/L) GAD autoantibody concentrations were serially diluted with the sample diluent prepared in Example 1. The obtained diluted samples were assayed for the antibody concentrations according to the method of the invention using the reagents and GAD antigen-immobilized plate prepared in Example 1.

The procedure (temperature, time, etc.) was as shown in Figure 2.

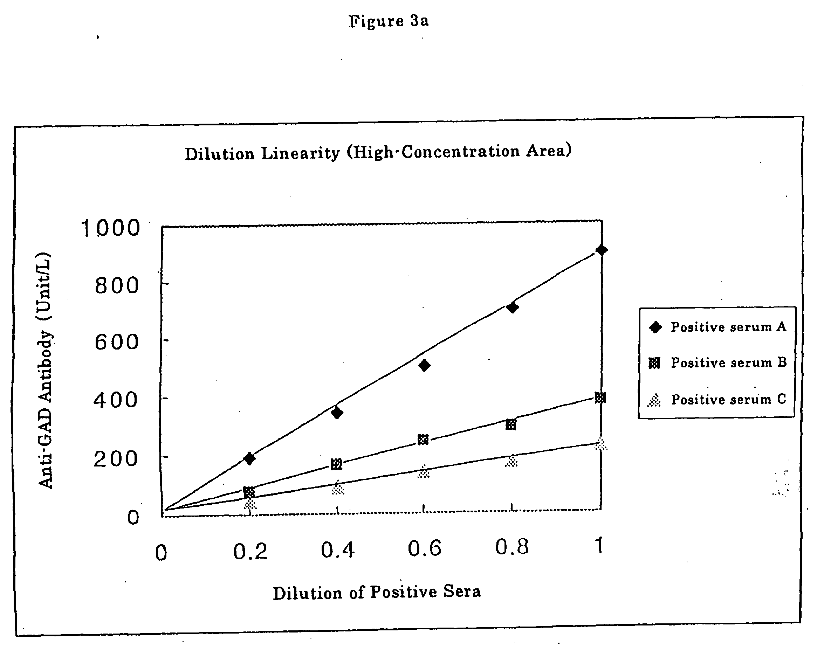

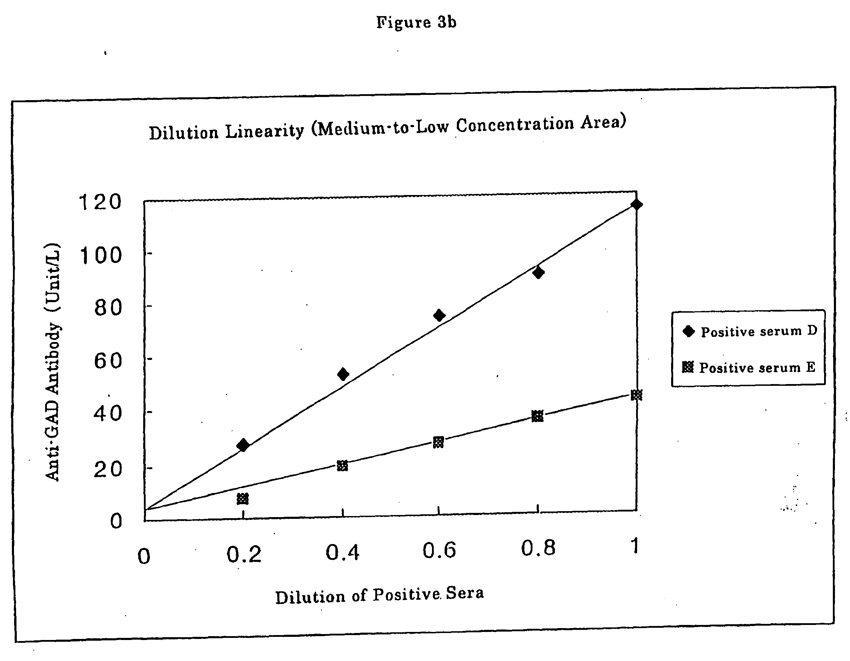

The results are shown in Figure 3. It was confirmed that there is a linearity through the origin between the dilution fold and the antibody concentration in all areas, from low to high values.

### Example 3: Test for Within-Day Precision

To examine the within-day precision of the method of the invention, the antibody concentrations of sera from two IDDM patients were assayed simultaneously at n=4 for three days using the reagents shown in Table 1 (for all reagents, three lots prepared on different days were used). The procedure was as shown in Figure 2.

The results are shown in Table 2. In all test results, the coefficient of variation (CV; %) was about 15% or less, demonstrating that the within-day precision was excellent.

**Table 2**

| Within-Day Precision | | | | | | | |
|---|---|---|---|---|---|---|---|
| Reagent Lot | | Sample | | | | | |
| | | Serum 1 | | | Serum 2 | | |
| | | 1st | 2nd | 3rd | 1st | 2nd | 3rd |
| 980824 | 1 | 21 | 28 | 27 | 49 | 54 | 61 |
| | 2 | 21 | 23 | 25 | 50 | 53 | 60 |
| | 3 | 20 | 22 | 27 | 52 | 57 | 60 |
| | 4 | 21 | 26 | 28 | 55 | 68 | 61 |
| | mean | 20.9 | 24.8 | 26.7 | 51.5 | 57.8 | 60.1 |
| | SD | 0.7 | 2.6 | 1.5 | 2.6 | 7.0 | 0.7 |
| | CV (%) | 3.27 | 10.63 | 5.51 | 5.13 | 12.08 | 1.14 |
| | N | 4 | 4 | 4 | 4 | 4 | 4 |
| 980825 | 1 | 23 | 28 | 31 | 51 | 57 | 62 |
| | 2 | 27 | 22 | 27 | 48 | 50 | 64 |
| | 3 | 24 | 22 | 25 | 49 | 52 | 67 |
| | 4 | 26 | 26 | 26 | 49 | 57 | 81 |
| | mean | 25.2 | 24.6 | 27.4 | 49.1 | 54.2 | 68.5 |
| | SD | 1.9 | 2.8 | 2.6 | 1.5 | 3.6 | 8.6 |
| | CV (%) | 7.66 | 11.31 | 9.46 | 3.05 | 6.59 | 12.50 |
| | N | 4 | 4 | 4 | 4 | 4 | 4 |
| 980826 | 1 | 22 | 28 | 29 | 47 | 46 | 57 |
| | 2 | 17 | 27 | 27 | 50 | 47 | 55 |
| | 3 | 20 | 23 | 27 | 50 | 52 | 62 |
| | 4 | 20 | 24 | 27 | 57 | 51 | 74 |
| | mean | 19.6 | 25.2 | 27.2 | 51.1 | 49.1 | 61.9 |
| | SD | 2.1 | 2.4 | 0.9 | 4.3 | 3.2 | 8.5 |
| | CV (%) | 10.91 | 9.65 | 3.38 | 8.35 | 6.42 | 13.69 |
| | N | 4 | 4 | 4 | 4 | 4 | 4 |

| | | | | | | | |
|---|---|---|---|---|---|---|---|
| *In the table, the units are in Unit/L, except for CV (%) and N. | | | | | | | |

### Example 4: Test for Day-to-Day Precision

To examine the day-to-day precision of the method of the invention, the antibody concentrations of sera samples of three IDDM patients were simultaneously assayed for six days using the reagents and GAD antigen-immobilized plate prepared in Example 1. The procedure was as shown in Figure 2.

The results are shown in Table 3. Calculating the CV (%) for the six measurements over 6 days for each sample showed that the CVs (%) for samples A, B, and C were 10% or less in all cases, demonstrating that the reproducibility was excellent.

**Table 3**

| Day-to-Day Precision for Six Days | | | | |
|---|---|---|---|---|
| Day | Sample A | Sample B | Sample C | Unit |
| 1 | 25 | 155 | 365 | (Unit/L) |
| 2 | 29 | 155 | 336 | |
| 3 | 29 | 155 | 349 | |
| 4 | 29 | 181 | 340 | |
| 5 | 29 | 168 | 340 | |
| 6 | 25 | 181 | 361 | |
| mean | 28.0 | 165.9 | 348.6 | |
| SD | 2.2 | 12.4 | 12.2 | |
| CV (%) | 7.75 | 7.47 | 3.49 | |

### Example 5: Effects of contaminants

The reagents and GAD antigen-immobilized plate prepared in Example 1 were used for the experiments. Human sera containing 40 Unit/L and 100 Unit/L an anti-GAD antibody were used as the samples. During the primary reaction, 250 mg/dl and 500 mg/dl hemoglobin, 20 mg/dl and 40 mg/dl bilirubin, 50 mg/dl and 100 mg/dl ascorbic acid, and chyle of Formazine turbidity 1500 and 3000 were present at the same time to determine the effects of these substances on the assay of anti-GAD antibody amounts.

The procedure was as shown in Figure 2.

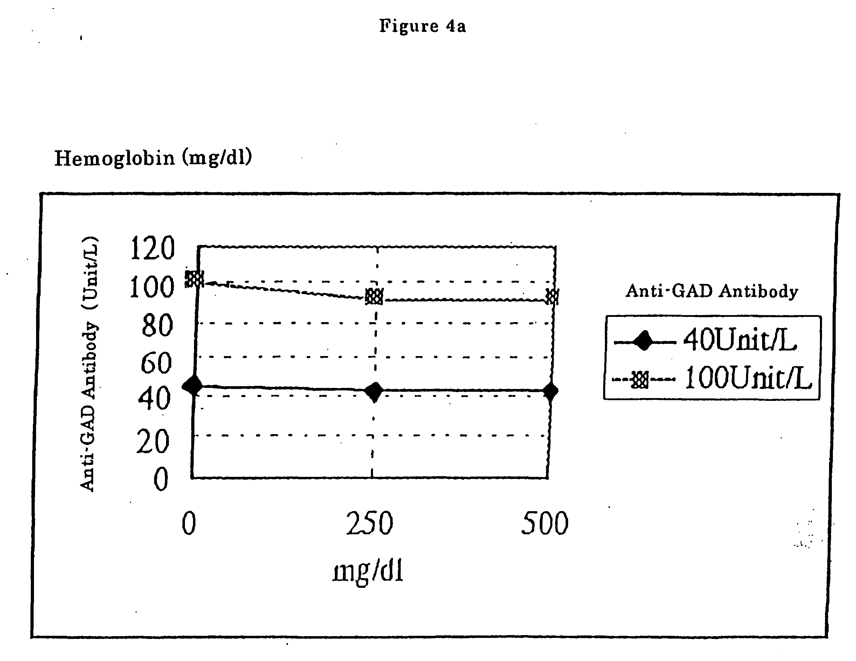

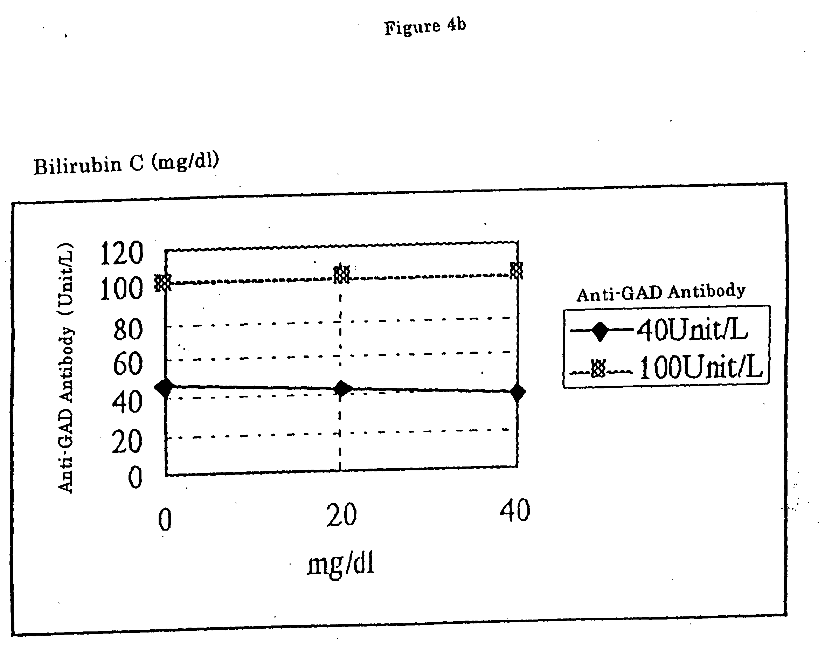

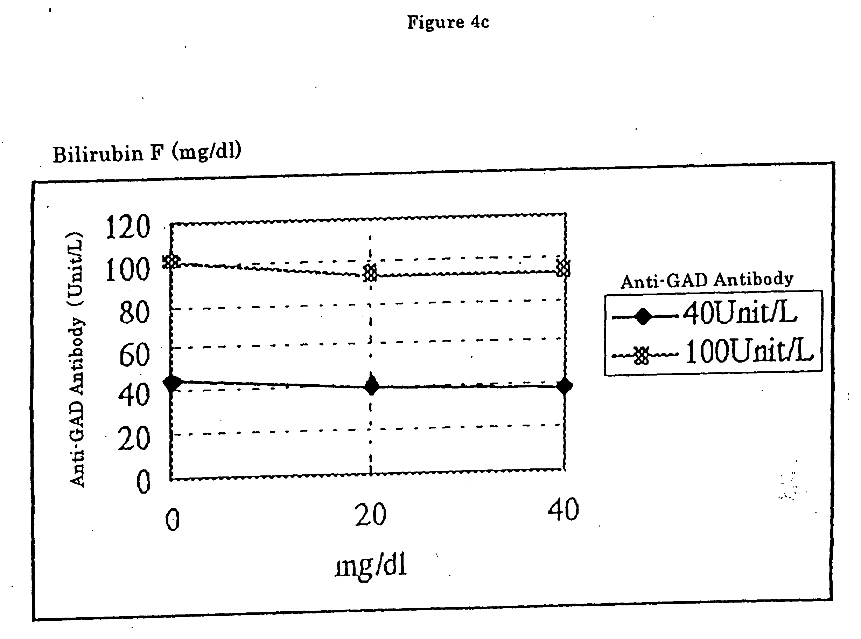

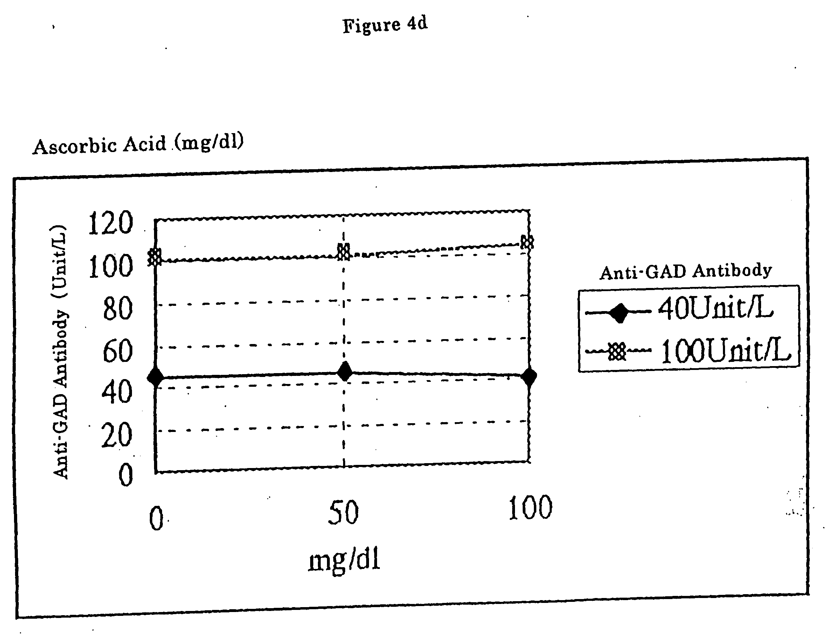

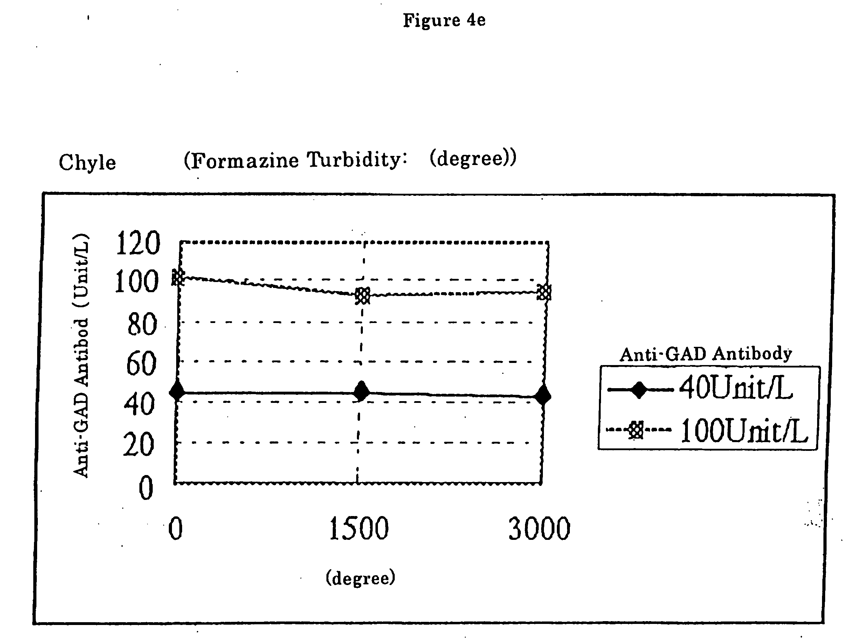

The results are shown in Figure 4. No effect of the contaminants on the assay for anti-GAD antibody amount was observed with 500 mg/dl or less of hemoglobin, 40mg/dl or less of bilirubin F or C, 100 mg/dl or less of ascorbic acid, or a Formazine turbidity of 3000 or less of chyle.

### Example 6: Determination of the detection sensitivity

A human anti-GAD antibody assay based on the chemiluminescence method of the invention was conducted using the reagents and GAD antigen-immobilized plate prepared in Example 1 in the procedure as shown in Figure 2 to obtain the detection sensitivity. Diluted samples containing low concentrations of anti-GAD antibodies were assayed simultaneously at n=3 for each concentration, the ±3SD for each sample calculated, and then the concentration of the human anti-GAD autoantibody that had the ±3SD range that did not overlap with that for the 0 Unit/L sample was confirmed.

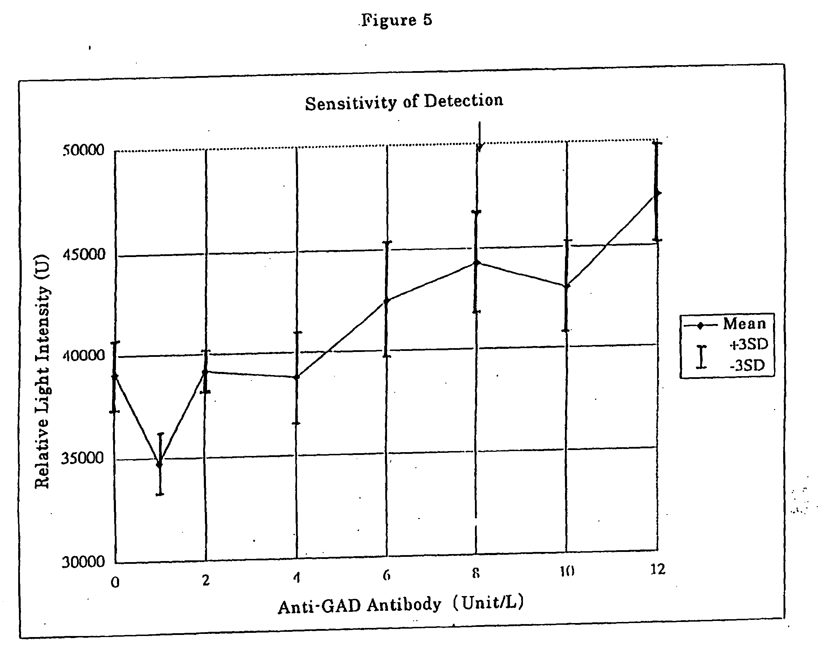

The results are shown in Table 4 and Figure 5 below. The human anti-GAD antibody concentration that had the ±3SD range that did not overlap with that for the 0 Unit/L sample was 8 Unit/L or more.

**Table 4**

| Detection Sensitivity | | | |
|---|---|---|---|
| Anti-GAD Antibody (Unit/L) | Mean | +3SD | -3SD |
| 0 | 39045.8 | 40780.9 | 37310.6 |
| 1 | 34721.8 | 36175.7 | 33267.8 |
| 2 | 39153.5 | 40188.3 | 38118.7 |
| 4 | 38768.8 | 41031.1 | 36506.4 |
| 6 | 42512.3 | 45352.2 | 39672.3 |
| 8 | 44278.0 | 46730.6 | 41825.4 |
| 10 | 43022.0 | 45251.6 | 40792.4 |
| 12 | 47464.3 | 49779.7 | 45148.8 |
| (Unit: Relative light intensity (U)) | | | |

### Example 7: Correlation between the measurement by the present method and that by radioactive immunoprecipitation

The human anti-GAD antibodies in IDDM patient sera were measured based on the chemiluminescence method of the invention by using the reagents and GAD antigen-immobilized plate prepared in Example 1. For the same samples, human anti-GAD autoantibodies were also measured by using a conventional human anti-GAD autoantibody assay kit "GAD Ab COSMIC" (imported and distributed by Cosmic Corporation) based on the radioactive immunoprecipitation principle. The assay by the method of the invention was conducted by the procedure as shown in Figure 2. GAD Ab COSMIC was used according to the manufacturer's instructions.

A diagram with a plot of the data by these two assays is shown in Figure 8. The vertical axis indicates the data by the method of the invention (ELISA) and the horizontal axis indicates the data by a conventional method (RIA); note that the antibody concentrations are uniformly expressed by the unit used in "GAD Ab COSMIC," i.e., Unit/mL. The correlation coefficient R was 0.954, demonstrating a high correlation between both series of measurements.

## Claims

1. A method for detecting or measuring an anti-GAD antibody comprising contacting a sample with a carrier on which a recombinant GAD antigen is immobilized, binding said antigen and said antibody in the sample to form a complex, and detecting said complex by a chemiluminescent dioxetane having a group that can be cleaved with an enzyme and an antibody labeled with an enzyme that can cause chemiluminescence by cleaving said group.

2. The method according to claim 1, wherein the labeled antibody is an antibody that can bind human anti-GAD antibody or an antibody that can bind a recombinant GAD antigen.

3. The method according to claim 1, wherein the labeled antibody is an anti-human IgG antibody.

4. The method according to claim 1, wherein the recombinant GAD antigen is a recombinant hybrid GAD67/65 antigen.

5. The method according to claim 1, wherein the enzyme is an alkaline phosphatase and the chemiluminescent dioxetane is a dioxetane that has a phosphate group and produces chemiluminescence by the cleavage of the group.

6. The method according to claim 1, wherein the chemiluminescence dioxetane is a dioxetane expressed by the formula: 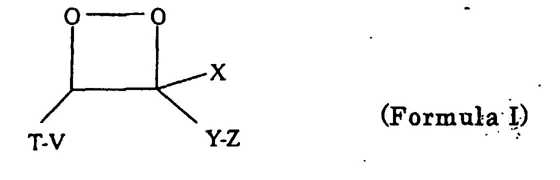 (wherein T is one or more of C [carbon number] 1 to C7 alkyls, C6 to C12 cycloalkyls which may be substituted with a heteroatom group, or polycycloalkyls which have a spiro-union with the four-member moiety of a dioxetane; V is a hydrogen or group that can be cleaved with an enzyme; Y is a fluorescent chromophore; X is a hydrogen, linear, or branched C1 to C7 alkyl, linear or branched C1 to C7 heteroalkyl, an aryl having at least one ring, a heteroaryl having at least one ring, C3 to C7 cycloalkyl, a C2 to C7 heterocycloalkyl, an aralkyl having at least one ring, an alkylaryl having at least one ring, or a group that can be cleaved with an enzyme; Z is a hydrogen, a hydroxyl group, or a group that can be cleaved with an enzyme; and at least one of V, X, and Z is a group that can be cleaved with an enzyme).

7. The method according to claim 1, wherein the chemiluminescent dioxetane is a dioxetane expressed by the formula: 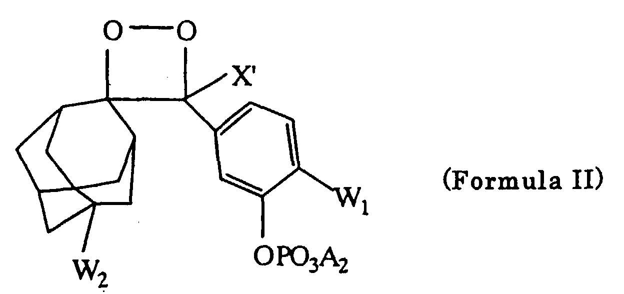 (wherein X' is a methoxy, ethoxy, propoxy, or butoxy; W₁ is a hydrogen or halogen; W₂ is a hydrogen, halogen, methoxy, isopropyloxy, or normal butyloxy; W₁ and W₂ may be the same or different; and A is an alkali metal).

8. The method according to claim 1, wherein the chemiluminescent dioxetane is disodium 2-chloro-5-(4-methoxyspiro{1,2-dioxetane-3,2'-(5'-chloro)-tricyclo[3.3.1.1^{3,7}] decan}-4-yl)-1-phenyl phosphate of the formula: 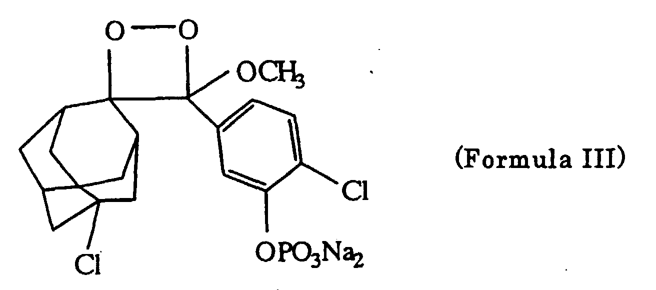 or disodium 3-(4-methoxyspiro{1,2-dioxetane-3,2'-tricyclo [3.3.1.1^{3,7}] decan}-4-yl) phenyl phosphate of the formula:

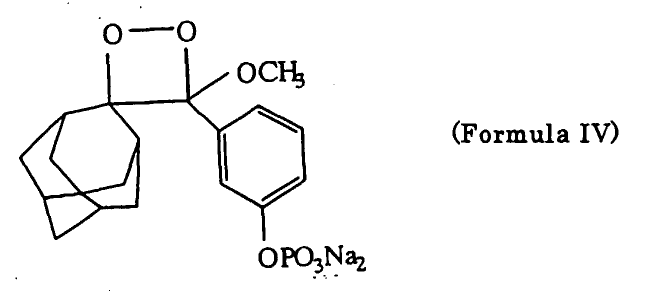

9. A kit for detecting an anti-GAD antibody comprising a carrier on which an recombinant GAD antigen is immobilized, a chemiluminescent dioxetane having a group that can be cleaved by an enzyme, and an antibody labeled with an enzyme that can cause chemiluminescence by cleaving said group.

10. The kit according to claim 9, wherein the labeled antibody is an antibody that can bind a human anti-GAD antibody or an antibody that can bind a recombinant GAD antigen.

11. The kit according to claim 9 wherein the labeled antibody is an anti-human IgG antibody.

12. The kit according to claim 9 wherein the recombinant GAD antigen is a recombinant hybrid GAD67/65 antigen.

13. The kit according to claim 9 wherein the enzyme is an alkaline phosphatase and the dioxetane has a phosphate group and causes chemiluminescence upon the cleavage of the group.

14. The kit according to claim 1, wherein the chemiluminescent dioxetane is a dioxetane expressed by the formula: 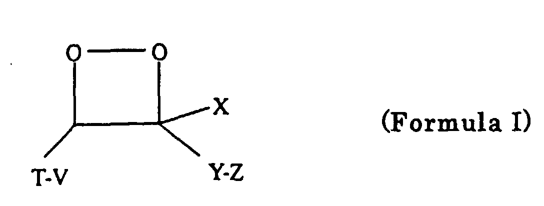 (wherein T is one or more of C [carbon number] 1 to C7 alkyls, C6 to C12 cycloalkyls which may be substituted with a heteroatom group, or polycycloalkyls which have a spiro-union with the four-member moiety of a dioxetane; V is a hydrogen or group that can be cleaved with an enzyme; Y is a fluorescent chromophore; X is a hydrogen, linear, or branched C1 to C7 alkyl, linear or branched C1 to C7 heteroalkyl, an aryl having at least one ring, a heteroaryl having at least one ring, C3 to C7 cycloalkyl, a C2 to C7 heterocycloalkyl, an aralkyl having at least one ring, an alkylaryl having at least one ring, or a group that can be cleaved with an enzyme; Z is a hydrogen, a hydroxyl group, or a group that can be cleaved with an enzyme; and at least one of V, X, and Z is a group that can be cleaved with an enzyme).

15. The kit according to claim 1, wherein the chemiluminescent dioxetane is a dioxetane expressed by the formula: 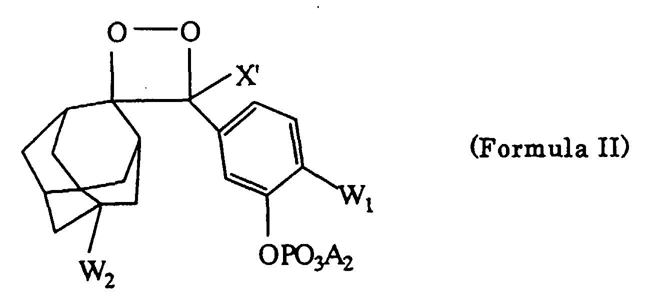 (wherein X' is a methoxy, ethoxy, propoxy, or butoxy; W₁ is a hydrogen or halogen; W₂ is a hydrogen, halogen, methoxy, isopropyloxy, or normal butyloxy; W₁ and W₂ may be the same or different; and A is an alkali metal).

16. The kit according to claim 1, wherein the chemiluminescent dioxetane is disodium 2-chloro-5-(4-methoxyspiro{1,2-dioxetane-3,2'-(5'-chloro)-tricyclo[3.3.1.1^{3,7}] decan}-4-yl)-1-phenyl phosphate of the formula: 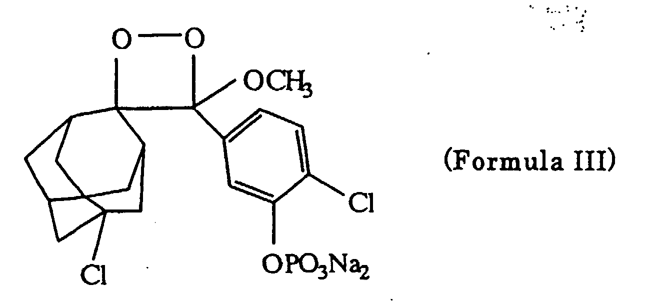 or disodium 3-(4-methoxyspiro{1,2-dioxetane-3,2'-tricyclo [3.3.1.1^{3,7}] decan}-4-yl) phenyl phosphate of the formula:

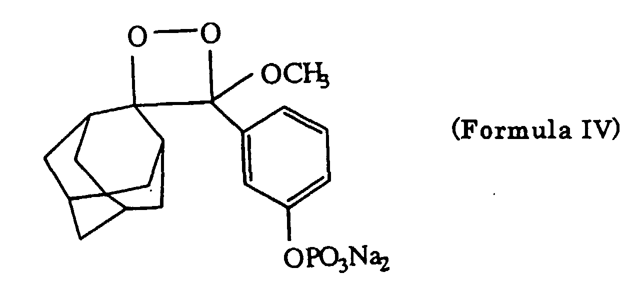

17. A kit for measuring a human anti-GAD antibody, comprising a carrier on which a recombinant hybrid GAD67/65 antigen is immobilized, a dioxetane having a phosphate group that can be cleaved with an alkaline phosphatase and causing chemiluminescence by the cleavage of the group, and an alkaline phosphatase-labeled anti-human immunoglobulin G antibody.
